(19) Europäisches Patentamt
European Patent Office
Office européen des brevets

(11) EP 0 606 356 B1

## (12) EUROPEAN PATENT SPECIFICATION

(45) Date of publication and mention
of the grant of the patent:
**03.06.1998 Bulletin 1998/23**

(21) Application number: **92921118.3**

(22) Date of filing: **29.09.1992**

(51) Int. Cl.$^6$: **A61B 5/028**, A61B 5/00

(86) International application number:
**PCT/US92/08263**

(87) International publication number:
**WO 93/06776 (15.04.1993 Gazette 1993/10)**

(54) **A DIAGNOSTIC CATHETER WITH MEMORY**

DIAGNOSTISCHER KATHETER MIT SPEICHERN

CATHETER DE DIAGNOSTIC AVEC MEMOIRE

(84) Designated Contracting States:
**DE FR GB**

(30) Priority: **01.10.1991 US 769536**

(43) Date of publication of application:
**20.07.1994 Bulletin 1994/29**

(73) Proprietor:
**INTERFLO MEDICAL, INC.
Plano, TX 75074 (US)**

(72) Inventors:
• **YELDERMAN, Mark, L.
Plano, TX 75093 (US)**
• **QUINN, Michael, D.
Plano, TX 75093 (US)**

(74) Representative:
**MacGregor, Gordon et al
Eric Potter Clarkson,
Park View House,
58 The Ropewalk
Nottingham NG1 5DD (GB)**

(56) References cited:
**EP-A- 0 221 357          EP-A- 0 378 234
EP-A- 0 417 781          US-A- 4 240 441
US-A- 4 407 298          US-A- 4 418 392
US-A- 4 481 804**

**Description**

**BACKGROUND OF THE INVENTION**

**Field of the Invention**

The present invention relates to a thermodilution catheter assembly for directly measuring temperature changes in a physiological medium of a patient, the assembly comprising a thermodilution catheter having a heating element, for heating the physiological medium.

Thermodilution catheters have been constructed such that thermistors can be mounted directly on the catheter, either at the tip, on the surface, or within the catheter body, for measuring temperature and sending information directly to a monitor or display device.

However, for particular catheter-mounted thermistors, certain errors are present. Some errors are inherent in the design of the thermistor; some are caused by variations in the thermistor as a result of manufacturing processes; some are caused by changes in the thermistor due to ageing or use; and some are patient specific. Although such errors can be measured, several practical problems arise. For example, although design or manufacturing errors can be measured for each individual thermistor, that information must be conveyed to either the end user or to a monitor or measuring device so that the errors may be compensated. For example, Lentz *et al* describe in US Patent No 4407298 a connector for a thermodilution catheter which joins the catheter to an output computer. However, the device of Lentz *et al* simply uses individual "bit" lines, each of which can be either open or closed so that four different coded states reflecting the size of the catheter are possible, and does not relay information about the thermistor to the output computer.

While Lentz *et al* do not describe that information about the thermistor may be contained on the catheter, other prior art catheter sensors utilize a memory unit which is connected to the sensors and to signal processing circuitry. For example, Meinema describes in US Patent No 4858615 an integral sensor and memory combination unit where information regarding the characteristics of the sensor or sensor-memory combination are permanently recorded in the memory and the sensor and memory are indissolubly coupled together. The recorded information (such as data for linearizing the sensor outputs) is automatically read and retrieved by separate electronic processing circuitry. However, the system of Meinema is described only for transducers which receive naturally occurring physiological parameters and is not described for measuring responses to heat from a heating element. In addition, Meinema corrects the transducer responses for both amplitude and offset and is concerned only with displaying a corrected physiological parameter. As a result, Meinema does not consider correcting or modifying the transducer for calculation, estunation, or computation of derived measurements. Furthermore, Meinema gives no consideration to correcting, modifying, compensating for heat from a heating element. It is thus desirable to provide a thermodilution catheter that is expanded to include this capability.

Non-catheter based measuring systems frequently have provided correcting means comprising memories for storing correction data. For example, Hata describes in US Patent No 4418392 a measuring device having a measurement correcting module with a memory unit for storing correction data which is used to correct digitized transducer data. However, this system requires the measured data to be altered at the analog to digital converter. It is desired that such modifications of the raw data be avoided to ensure accuracy. Similarly, Baily describes in US Patent No 4446715 using correcting means responsive to calibration means for correcting the measured physical variable. This is done for pressure transducers which are not catheter based by using information from a ROM (Read Only Memory) to correct the transducer output without any incorporation of the information into a microprocessor program. However, as with the system of Hata, this system requires the raw data to be modified.

Other disclosures directed to calibration of non-catheter-based sensors using a memory device include US Patent No 4481804; US Patent No 4499547; US Patent No 4611304; US Patent No 4868476; and US Patent No 4942877. US Patents Nos 4770179, 4700708 and 4621643 disclose an oximeter with a calibration system. EP-A-0221357 discloses such a system in a catheter. US Patent No 4856530 describes a calibration system using a capacitor to store the calibration information.

In addition, in a thermodilution catheter although errors which arise once the thermistor is in use, either because of ageing or other processes of the thermistor or because of patient physiological variations, can be measured by *in vivo* or patient calibration tests, again the results must be retained by the measuring device or monitor for display to the end user. Moreover, a more serious problem is that the thermistor, once inserted in a patient, cannot be removed. Rather, the inserted thermistor must move with the patient. Nevertheless, when the patient is moved from one critical care environment to another, such as from the operating room to the intensive care unit, the monitoring equipment is often not moved, but rather the catheter is disconnected from the original monitor and reconnected to another monitor in the new location. Such disconnection typically results in the loss of thermistor specific or patient specific information or requires the operator to re-enter the information, resulting in increased work, frustration, and reduction in quality of patient care. Similar problems arise when heating element information is lost.

Another problem for catheter manufacturers is that generally the catheter is relatively simple in proportion to the complexity of the computing, calibration and display devices, yet the profits are made from the sale of the catheters, not the monitors. As a result, even though a manufacturer may develop, manufacture, and sell the catheter and display device as a system, the catheter can be easily replicated by a competitor and manufactured and sold without the display device, resulting in a significant loss of profits for the original manufacturer. This can be somewhat prevented if the catheter and display device have some mechanism by which a competing manufacturer may be prevented from copying the catheter alone and selling it in place of the original catheter. A suitable mechanism of this type is desired.

Previous inventors have addressed this problem by designing various types of devices for encoding transducer factors for calibration. For example, US-A-4303984 discloses placing in a common connector a ROM, shift register and other sensor electronics powered by a power supply which is also included in the same connector. In this device, when the ROM information is desired, the information is "clocked" from the ROM and is combined or superimposed onto the raw sensor electronics. However, such an arrangement is unduly complicated and expensive for use in a thermodilution catheter of the type to which the present invention is directed. A simpler and less expensive alternative is desired.

Accordingly, it is desired to provide a thermodilution catheter with memory which can retain information specific to heating element calibration, and preferably also patient specific calibration data, historical patient data and the like. It is also preferably that this information be coded to prevent unauthorised access.

Accordingly, the present invention provides a thermodilution catheter assembly as set out in Claim 1.

The preamble of Claim 1 is based on US-A-4240441, which discloses a thermodilution catheter assembly for directly measuring temperature changes in a physiological medium of a patient, the assembly comprising a thermodilution catheter having a heating element for heating the physiological medium.

The memory may retain information specific to factor calibration, patient calibration data, patient historical data, encoded data and the like. The catheter may be used with the processing circuitry of US Patent No 5146414. In accordance with the techniques set forth in this patent, cardiac output is measured which requires the transfer of indicator from a heater filament to the flowing blood and the measurement of the response at a distal thermistor.

When the memory contains patient specific information which can be accessed by the processing system to which the catheter assembly is connected for processing. Preferably, the memory is selected such that disconnection of the device from the processing system does not cause values stored in the memory to be lost so that the patient specific information need be reentered into the memory when the device is reconnected to the same or another processing system. Also, in order to prevent piracy, it is preferred that the stored data be encoded.

The memory of the invention may be disposed at different locations within the catheter assembly. For example, the memory may be disposed within the body of the catheter, in an area adjacent one of the thermocouple or thermistor and heating element; or in a connector connected to a proximal end of the catheter assembly for allowing communication with the processing system, which may be a conventional external processing system or computer. Such a connector preferably comprises leads which are connected to the memory so as to allow access to contents of the memory by the external processing system connected to the catheter.

The catheter may be designed for single patient use or multiple patient use.

The connector has leads that are connected such that the external processing system can write calibration information to the memory of the catheter during operation for in vivo calibration. This information may then be used during processing of the detected data to make necessary corrections or modifications to the transducer outputs or the subsequent computations using the raw information received from the transducers.

During operation, the external processing system may access the patient specific information in the memory via the connector leads so that the memory may provide historical patient information to the external processing system for display as trending data of the patient. This information is maintained such that even when the catheter assembly is disconnected from the external processing system the patient's historical data can be later retrieved when the catheter assembly is reconnected to the same or another external processing system. For this purpose, the catheter assembly may further comprise a battery located in proximity of the memory for providing power to the memory when the memory is not connected to the external processing system. In addition, the calibration information and patient specific information are preferably encoded in accordance with a proprietary code stored in the memory. This proprietary code may then be read by the external processing system to determine whether the catheter assembly is supplied by a particular manufacturer prior to conducting further processing. Preferably, the proprietary code is a binary code stored in the memory and is accessed by the external processing system and used thereby to decode the encoded calibration information and encoded patient specific information.

The memory may further contain catheter identification information including manufacture date, batch number, sterilization date, expiration date, catheter transducer number and type, manufacturer's name and address and any other unique identification or process information. In addition, the memory may also contain a computer program, a computer program segment, a software subroutine and computer memory addresses which can be read by the external processing system and used thereby to verify, correct, or modify the processing of the catheter transducer information. In such an embodiment, the software of the catheter memory and the external processing system together form a unique software combination such that system operation cannot occur without the two software pieces together. This assures that only catheter memories programmed by particular manufacturers can be used with a particular processing system. For this purpose, the memory may further contain a proprietary code which is read to determine whether the catheter assembly is supplied by a particular manufacturer.

## BRIEF DESCRIPTION OF THE DRAWINGS

The above and other objects and advantages of the invention will become more apparent and more readily appreciated from the following detailed description of the presently preferred exemplary embodiment of the invention taken in conjunction with the accompanying drawings, of which:

FIGURE 1 illustrates a calibration circuit having a memory in accordance with a presently preferred embodiment of the invention.
FIGURE 2 illustrates in more detail the connections of the memory of FIGURE 1 for the case where the memory is a CAT93C46 1 Kbit serial EEPROM.
FIGURES 3 and 4 respectively illustrate top and side views of the catheter connector assembly at the proximal end of a catheter having a memory in accordance with the invention.
FIGURE 5 illustrates an end view of a connector cover for covering the catheter connector assembly shown in FIGURE 3.

## DETAILED DESCRIPTION OF THE PREFERRED EMBODIMENT

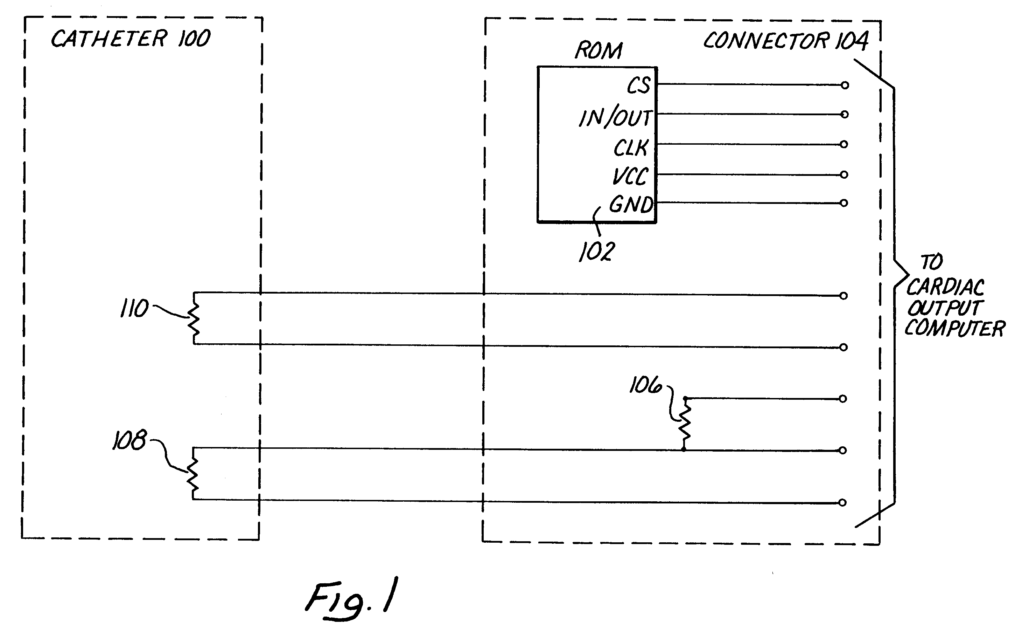

A system with the above-mentioned beneficial features in accordance with presently preferred exemplary embodiments of the invention will be described below in detail with reference to FIGURES 1-5.

In the calculation of cardiac output using a thermodilution catheter and an associated processing system, it is necessary to know certain properties about the distal measuring transducer, such as a thermistor or thermocouple, and the heating filament efficiency, for in the manufacturing process it is difficult to produce either thermistors or thermocouples or heating filaments which uniformly have the same properties. Thus, to reduce the errors which would be introduced into the calculation of cardiac output due to these variances, it is necessary to calibrate or measure the physical properties of both the thermistor or thermocouple and the heating filament. Since in a clinical environment each cardiac output computer may be attached over time to various pulmonary artery catheters and to eliminate the need for the user to manually transcribe these calibration numbers to the computer, a coding technique has been developed to pass the calibration information.

Prior art thermodilution catheters and pulse oximeter sensors have used resistors to code the values for thermistors or LEDs. For example, New, Jr. et al. in the aforementioned U.S. Patent No. 4,700,708 use a resistor to calibrate LED wavelengths on a pulse oximeter. However, the present inventors know of no previous attempt to code the filament calibration for transferring the calibration information of the heating filament solely or the calibration information of the heating filament and thermistor or thermocouple together. Thus, calibration of the heating element may be conducted by measuring the heater resistance at a known temperature. The catheter assembly can then use the previously calibrated thermistor or thermocouple and a built-in ohm meter to establish a calibrated reference point for the heater element. This approach has the advantage of calibrating the heater immediately prior to use in a patient at the patient's body temperature. Such an accurate calibration of heater resistance and temperature is necessary to accurately monitor heater temperature to insure patient safety.

The calibration circuit of the assembly may include passive electronic components such as resistors, inductors and capacitors such that the value of the components correspond to a particular calibration value or number according to a predetermined table. On the other hand, active electronic components including numerous nonlinear components may be used such that a particular performance corresponds to a particular calibration number or value. Such calibration information is preferably stored in a memory component such as a ROM (Read only Memory), RAM (Random Access Memory), nonvolatile memory devices or other types of volatile or nonvolatile memory or digital devices or any desired size. The calibration information preferably includes codes that represent the filament resistance, filament efficiency, and other parameters. If properly selected, one or more electronic components may be used to encode the calibration information of the thermistor or thermocouple, such as its $\beta$ value, and the filament resistance, filament efficiency and other parameters.

Thus, the calibration information for both the thermistor or thermocouple and the heating filament may be encoded by one or more active or passive electronic components or these values may be stored in a suitable memory device. The cardiac output computer may then decode this information and incorporate it into the calculation of cardiac output, for example. However, this step may be eliminated if the actual appropriate software is contained in the catheter itself. For example, a memory device such as a ROM may be contained in the catheter with a portion of the software utilized by the cardiac output computer resident within it. Such information might include program segments or historical patient data. Thus, when the catheter is connected to the cardiac output computer, prior to the beginning of processing for determining the cardiac output, the software or program segment contained in the catheter memory device (ROM or RAM) may be transferred to the main software program of the cardiac output computer. This feature of the invention also provides an additional safety feature, for the cardiac output computer will not start until it has transferred the program segment and incorporated this segment into its own program.

The calibration circuitry of the type just described can be seen by way of example in FIGURE 1. As should be apparent to one of ordinary skill in the art, the calibration circuit of FIGURE 1 is quite different from that used in typical prior art thermodilution catheters. In particular, classic thermodilution catheters use calibration resistances which are connected to form one-half of a bridge circuit with the thermistor or thermocouple. In such devices, the reference resistor is selected to match the thermistor or thermocouple for a standard temperature. In this manner, compensation for variability in the thermistors or thermocouples may be achieved. However, by using the calibration circuit whereby a RAM or ROM containing calibration data is included within the connector of the catheter, such a reference resistor for calibration purposes is not needed. Such a memory for use with a thermodilution catheter 100 is shown as memory 102 of connector 104 in FIGURE 1.

Preferably, the software module referred to above is stored in the memory 102 and includes such things as the format version for the calibration data, trademark information, historical patient data (such as cardiac output for the previous several hours) or whatever information is desired for controlling the cardiac output program. Thus, by placing the encoded calibration data within the memory 102 and placing the memory 102 on the catheter 100, the reference resistance 106 for the thermistor or thermocouple 108 may be eliminated. In addition, only a catheter having a memory 102 storing the necessary information for operating the program of the cardiac output computer may be used in conjunction with the cardiac output computer to obtain the desired calculation.

FIGURE 1 illustrates a method of enhancing the performance of a catheter by retaining factory calibration, factory identification, computer or monitor specific software program segments, patient specific calibration information, and patient historical information in the catheter which is not lost when the catheter is disconnected from the computer, monitor or other display device, as when the patient is moved.

In particular, the catheter of the invention contains in the body, connector, or some other aspect of the catheter a memory 102 which can be accessed by any of a variety of means when the catheter is connected to an external processing device such as a cardiac output computer. The memory 102 is either of a volatile or nonvolatile type such that when the memory 102 is not connected to the external processing device the memory contents are not lost. In addition, the external processing device is preferably allowed, when connected to the catheter 100 and consequently to the memory 102, to address any byte of the memory 102 and to either read or write to the byte at that address. In addition, the relevant information can be written to the appropriate address of the memory 102 during the portion of the manufacturing process during which the calibration data is measured.

In a preferred embodiment of the invention, different segments of the memory 102 may contain the following information segments:

1. A catheter unique serial number;
2. Manufacturing identification data, such as calibration, manufacture, sterilization and ship date or any other date and time information relevant to the catheter 100;
3. A software program segment which is not integral to the catheter 100 or to any aspect of the catheter 100 or heating element 110, but is instead program information, such as a subroutine, which is incorporated into the software program of the display device;
4. A unique security code which allows the monitor to identify a catheter which has been manufactured by the manufacturer of the monitor or a competing manufacturer; and
5. Manufacture and thermal calibration information about the heating element 110 which is the part of the catheter 100 used to introduce heat into the flowing blood for the thermodilution measurement. Such information could contain, for example, filament nominal electrical resistance, heat transfer coefficient, thermal mass, filament composition and coefficient of resistance.

Of course, in view of the present disclosure, those skilled in the art will appreciate that other desirable information may be kept in the memory 102 as well.

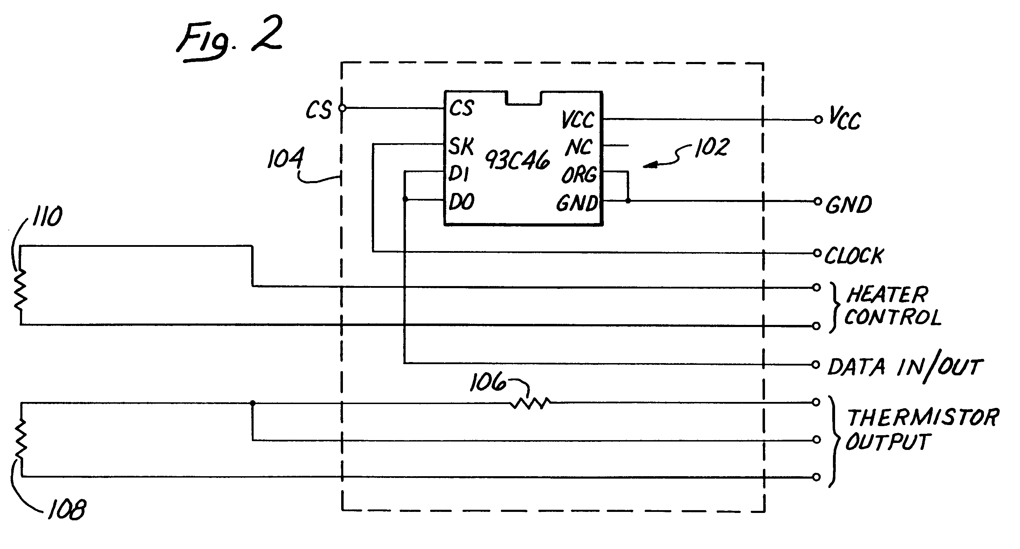

The present invention will now be described in more detail with respect to Figures 2-5.

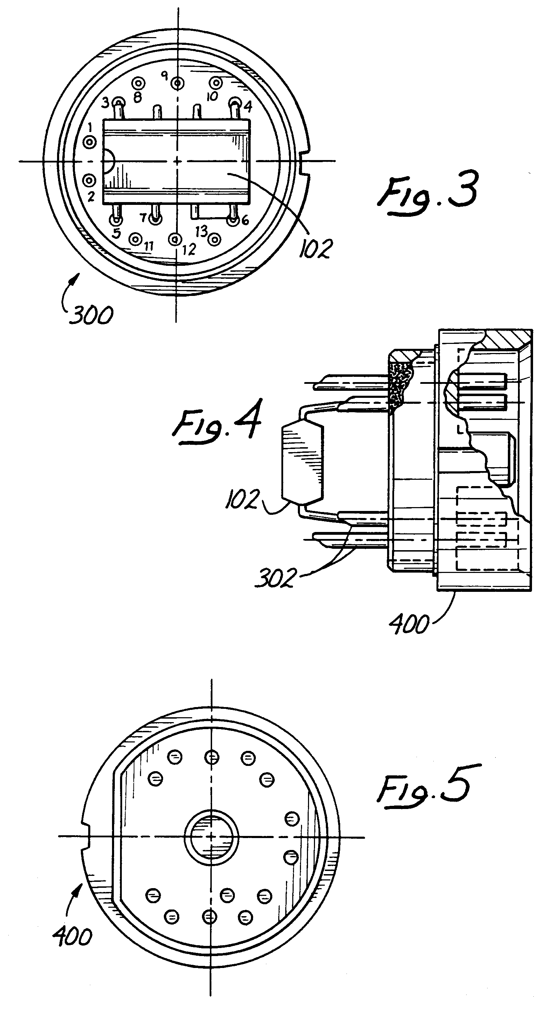

Figure 2 illustrates a schematic for a catheter memory 102 in accordance with a preferred embodiment of the invention. As shown, a standard thermistor/resistor bridge catheter assembly having reference resistor 106 and thermistor 108 may be used as in the embodiment of Figure 1 to measure blood temperature. Catheter memory 102 is also provided and is connected as shown to include a voltage supply lines (VCC), clock lines (SK), data lines (DI and DO), and a ground (GND). In the presently preferred embodiment, a CAT93C46 1 Kbit serial EEPROM is used as memory 102 and is connected as shown, where CS indicates "chip select", NC indicates "no connection" and ORG indicates "memory organization". As would be apparent to one skilled in the art, although only one address or "clock line" is shown, any number of lines can be used. Also, as shown in more detail in FIGURES 3 and 4, the address and data lines preferably go to a connector 300, and these address and data lines may be shared with other transducer's lines, which in the case illustrated are filament heater lines.

FIGURES 3-5 illustrate in more detail the catheter connector 300. As shown, the memory or chip 102 is mounted in the proximal end of the catheter at the connector 300. Connector pins 302 are attached to the pins of the memory chip 102 so as to allow the memory 102 to be accessed by an external processing device when the catheter connector 300 is plugged into the external processing device either directly or via a connecting cable. The catheter assembly may further include a connector cover 400 as shown in FIGURES 4 and 5 to protect the memory chip 102 from damage.

As noted above, in a preferred embodiment of the invention the memory 102 is a CAT93C46 1 Kbit serial EEPROM. A CAT93C46 memory device is organized in 64 registers of 16 bits (ORG pin at VCC) or 128 registers of 8 bits each (ORG pin at GND). Each register can be written or read serially by using the DI or DO pins. The CAT93C46 memory device is desirable since it is a CMOS EEPROM with floating gates, operates at 700 KHz, and is designed to endure 10,000 erase/write cycles and a data retention of 10 years. However, those skilled in the art will realize that other memory devices will satisfy the characteristics of the present invention.

The allocation and use of memory 102 will now be described. In particular, the algorithm used to encode and decode the data stored in the EEPROM of several models of thermodilution catheters will be described.

As noted above, the purpose of encoding the data in the catheter EEPROM is to make it more difficult to copy or counterfeit the catheters in which the present invention is used, such as the catheters described in the parent application. For this purpose, an algorithm is used to encode selected bytes of data within the catheter EEPROM. For example, in a preferred embodiment the first two (2) bytes of data in the EEPROM need not be encoded. This allows the software of the external processing device to read the security code in those bytes. This code is the basis of an encrypting/decrypting key for the remainder of the stored data. Several other bytes also need not be encoded (such as bytes 02 through 07) and preferably contain product information such as model number and serial number and the like which may also be read by the software of the external processing device. The remaining bytes are encoded and are initialized to contain the manufacturer's copyright notice and checksums (arithmetic 8-bit sums) which may be used by the security algorithm as shown in **TABLE 1** below.

The following algorithm is preferably utilized to encode or decode the stored data. First, the security code is read from bytes 00 and 01. This code may be, for example, 0314 Hex, but any 16-bit value is possible. The checksum in byte 127 is then read and ANDed with the security code. This result is then ANDed with the complement of the security code and shifted right four places. This forms the encryption/ decryption key. The data to be encrypted or decrypted is exclusive-ORed, on a word basis, with the key. The above may be illustrated by a simple C code expression as follows:

data ^= ((security_code & cksum) & ~security_code) >> 4;

Also, the information related to factory calibration of the catheter filament is preferably stored and read from byte 08. Of course, those skilled in the art will readily appreciate that many other types of known encoding schemes may be used. For example, the proprietary code may also be encrypted.

The data in a preferred embodiment of memory 102, after initialization, will thus appear as follows:

TABLE 1

| Byte | Function |
|---|---|
| 00 - 01 | Unencoded security code |
| 02 - 05 | Unencoded serial number |
| 06 | Unencoded layout byte |
| 07 | Unencoded model number |
| 08 | Encoded heater resistance |
| 09 - 32 | Encoded remaining data |
| 33 | Encoded checksum of above data |
| 34 | Zero byte |
| 35 - 38 | Longword, number of seconds since 1/1/70 |

TABLE 1 (continued)

| Byte | Function |
|------|----------|
| 39 | Checksum of all above bytes |
| 40 - 41 | Zero bytes |
| 42 - 82 | "Copyright (c) 1991 Interflo Medical, Inc." |
| 83 | Zero byte |
| 84 - 126 | Random uninitialized data bytes |
| 127 | Checksum of all above 127 bytes |

Then, for example, the data in the EEPROM, after patient data has been collected, will appear as follows:

TABLE 2

| Byte | Function |
|------|----------|
| 34 - 35 | Patient Weight |
| 36 - 37 | Patient Height |
| 38 | Reserved |
| 39 | Checksum of above five (5) bytes |
| 40 - 43 | Timestamp of 1st CO data point |
| 44 - 45 | Count of all CO data points in EEPROM |
| 46 - 109 | Last 64 CO data points at 15 minute intervals |
| 110 | Reserved |
| 111 | Checksum of bytes 40 through 110 |

This data is the "historical patient data" in a preferred embodiment, although other data may of course be collected.

After manufacture of the catheter assembly, the memory 102 may be accessed by an appropriate device to determine if the code stored in the memory 102 is the proper code. If this code is not the proper code, then it is known that the catheter assembly being checked is faulty or is an unauthorized copy. The tester then may choose to render the tested catheter non-functional or temporarily or permanently inoperative through any of a variety of means. In this manner, a mechanism is provided to insure that the catheter assembly being used is not an imitation catheter and to prevent such a catheter assembly from being inserted into the patient and connected to the monitor.

As described above, the information in the memory 102 is accessible and changeable by the external computing, calculation, display, or monitoring means in the field during clinical use. However, before the catheter memory 102 leaves the factory, some of information is preferably written to the catheter memory 102 including catheter and/or transducer test, calibration, or date information.

The memory 102 may have a small battery backup located on the connector 300 with the memory chip. Also, the memory 102 may be of any desired size and may be read only or read/write memory. In addition, the memory may be used alone or in combination with a variety of other components such as multiplexers, capacitors, resistors, operational amplifiers and the like. The memory 102 also may be combined directly with other electronic components such as amplifiers, resistors, capacitors, inductors, other memory units, multiplexers, shift registers, batteries, and the like and further may be combined either directly or through the connector leads to any or all catheter transducers. Furthermore, the memory 102 may reside on a removable sensor probe that fits within a lumen of the catheter or may be included in the catheter or connector in such a way that it is accessible not directly by the external processing system but rather by means of one of the internal transducers.

## Claims

1. A thermodilution catheter assembly for directly measuring temperature changes in a physiological medium of a patient, the assembly comprising a thermodilution catheter (100) having a heating element for heating the physiological medium,

characterised by a memory (102) which resides at a predetermined location on the thermodilution catheter, the memory containing coefficient of resistance information of the heating element and heat transfer coefficient information of the heating element, decodable by an external processing system for calibrating the resistance and temperature of the heating element.

2. The thermodilution catheter assembly of Claim 1 wherein the memory (102) contains thermal mass information for the heating element.

3. The thermodilution catheter assembly of Claim 1 or 2, wherein the memory (102) contains filament composition information for the heating element.

4. The thermodilution catheter assembly of any preceding claim, wherein the memory (102) contains patient specific information which is accessible by the external processing system.

5. The thermodilution catheter assembly of any preceding claim, wherein said predetermined location is within the body of said catheter (100).

6. The thermodilution catheter assembly of any preceding claim, wherein said predetermined location is an area adjacent said heating element.

7. The thermodilution catheter assembly of any preceding claim, wherein said catheter includes a thermistor or a thermocouple for measuring temperature changes in said physiological medium caused by said heating element.

8. The thermodilution catheter assembly of any preceding claim further comprising a battery located in proximity of said memory (102) for providing power to said memory when said memory is not connected to an external processing system.

9. The thermodilution catheter assembly of any preceding claim, wherein said memory (102) further contains catheter identification information including at least one of manufacture date, batch number, sterilization date, expiration date, heating element number and type and manufacturer's name and address.

10. The thermodilution catheter assembly of any preceding claim further comprising a connector (300) connected to a proximal end of said thermodilution catheter for connecting the assembly to the external processing system to allow said heating element to communicate with said external processing system, wherein said predetermined location is within said connector (300).

11. The thermodilution catheter assembly of Claim 10, wherein said connector (300) comprises leads (302) which are connected to said memory (102) so as to allow access to contents of the memory by said external processing system when connected to said catheter assembly.

12. The thermodilution catheter assembly of any one of Claims 1 to 11, wherein said memory (102) further contains at least one of a computer program, a computer program segment, a software subroutine and computer memory addresses which can be read by said external processing system and used thereby to verify, correct, or modify the processing of the heating element calibration information.

13. The thermodilution catheter assembly of any preceding wherein the memory (102) is connected to an external processing system.

14. The thermodilution catheter assembly of Claim 13, wherein the temperature changes measured by the thermodilution catheter assembly are used by said external processing system to measure cardiac output or flow of the physiological medium of said patient.

15. The thermodilution catheter assembly of Claim 13 or 14 when dependent from Claim 12, wherein said connector leads (302) are connected such that said external processing system can write calibration information for said patient or said heating element to said memory (102) during operation of said catheter assembly for *in vivo* calibration.

16. The thermodilution catheter assembly of any one of Claims 13 to 15 when dependent from Claim 11, wherein said external processing system accesses said patient specific information via said leads (302) and said memory (102) provides said patient specific data to said external processing system for display.

**Patentansprüche**

1. Wärmedilutionskatheteranordnung zur direkten Messung von Temperaturänderungen in einem physiologischen Medium eines Patienten, wobei die Anordnung einen Wärmedilutionskatheter (100) mit einem Heizelement zum Erwärmen des physiologischen Mediums unfaßt, gekennzeichnet durch einen Speicher (102), der sich an einer bestimmten Stelle auf dem Wärmedilutionskatheter befindet, und der Speicher Informationen über den Widerstandskoeffizienten des Heizelements und Informationen über den Wärmeübertragungskoeffizienten des Heizelements enthält, die durch ein externes Verarbeitungssystem zur Kalibrierung des Widerstandes und der Temperatur des Heizeiements dekodierbar Sind.

2. Wärmedilutionskatheteranordnung nach Anspruch 1, dadurch gekennzeichnet, daß der Speicher (102) Informationen zur thermisch wirksamen Masse für das Heizelement enthält.

3. Wärmedilutionskatheteranordnung nach Anspruch 1 oder 2, dadurch gekennzeichnet, daß der Speicher (102) Informationen zur Heizfadenzusammensetzung für das Heizelement enthält.

4. Wärmedilutionskatheteranordnung nach einem der vorhergehenden Ansprüche, dadurch gekennzeichnet, daß der Speicher (102) spezifische Informationen über den Patienten enthält, die über das externe Verarbeitungssystem zugänglich sind.

5. Wärmedilutionskatheteranordnnung nach einem der vorhergehenden Ansprüche, dadurch gekennzeichnet, daß die bestimmte Stelle innerhalb des Katheterkörpers (100) liegt.

6. Wärmedilutionskatheteranordnung; nach einem der vorhergehenden Ansprüche, dadurch gekennzeichnet, daß die bestimmte Stelle eine dem Heizelement benachbarte Zone ist.

7. Wärmedilutionskatheteranordnung nach einem der vorhergehenden Ansprüche, dadurch gekennzeichnet, daß der Katheter einen Thermistor oder ein Thermoelement zur Messung von durch das Heizelement verursachten Temperaturänderungen in dem physiologischen Medium umfaßt.

8. Wärmedilutionskatheteranordnung nach einem der vorhergehenden Ansprüche, dadurch gekennzeichnet, daß sie ferner eine in Nachbarschaft des Speichers (102) befindliche Batterie umfaßt, um den Speicher mit Energie zu versorgen, wenn der Speicher nicht mit einem externen Verarbeitungssystem verbunden ist.

9. Wärmedilutionskatheteranordnung nach einem der vorhergehenden Ansprüche, dadurch gekennzeichnet, daß der Speicher (102) ferner Informationen zur Identifizierung des Katheters enthält, die mindestens eine solche umfassen bestehend aus Herstellungsdatum, Chargennummer, Sterilisationsdatum, Verfalldatum, Heizelementzahl und -typ, und Name und Adresse des Herstellers.

10. Wärmedilutionskatheteranordnung nach einem der vorhergehenden Ansprüche, dadurch gekennzeichnet, daß er ferner ein Verbindustgselement (300) umfaßt, das mit einem proximalen Ende des Wärmedilutionskatheters verbunden ist, um die Anordnung mit dem externen Verarbeitungssystem zu verbinden, und es dadurch dem Heizelement zu ermöglichen, mit dem externen Verarbeitungssystem zu kommunizieren, wobei die vorbestimmte Stelle innerhalb des Verbindungselements (300) liegt.

11. Wärmedilutionskatheteranordnung nach Anspruch 10, dadurch gekennzeichnet, daß das Verbindungselement (300) Leitungen (302) unfaßt, die mit dem Speicher (102) verbunden sind, um über das externe Verarbeitungssystem Zugang zum Speicherinhalt zu haben, wenn dieses mit der Katheteranordnung verbunden ist.

12. Wärmedilutionskatheteranordnung nach einem der Ansprüche 1 bis 11, dadurch gekennzeichnet, daß der Speicher (102) ferner einen der Bestandteile bestehend aus einem Computerprogramm, einem Computerprogrammteil, einem SoftwareUnterprogramm und Computerspeicheradressen, enthält, die durch das externe Verarbeitungsprogramm gelesen werden können, und somit verwendet werden können, um die Verarbeitung der Information zur Kaiibrierung des Heizelements zu überprüfen` zu korrigieren oder zu modifizieren.

**13.** Wärmedilutionskatheteranordnung nach einem der vorhergehenden Ansprüche, dadurch gekennzeichnet, daß der Speicher (102) mit einem externen Verarbeitungssystem verbunden ist.

**14.** Wärmedilutionskatheteranordnung nach Anspruch 13, dadurch gekennzeichnet, daß die durch die Wärmedilutionskatheteranordnung gemessenen Temperaturänderungen von dem externen Verarbeitungsprogramm verwendet werden, um die Herzleistung oder den Fluß des physiologischen Mediums des Patienten zu messen.

**15.** Wärmedilutionskatheteranordnung nach Anspruch 13 oder 14, wenn abhängig vom Anspruch 12, dadurch gekennzeichnet, daß die Verbindungselementleitungen (302) so verbunden sind, daß das externe Verarbeitungssystem die Information zur kalibrierung für den Patienten oder das Heizelement wahrend des Betriebs der Katheteranordnung für eine in-vivo-Kalibrierung zum Speicher übertragen kann.

**16.** Wärmedilutionskatheteranordnung nach einem der Ansprüche 13 bis 15, wenn abhängig vom Anspruch 11, dadurch gekennzeichnet, daß das externe Verarbeitungssystem zu den spezifischen Informationen über den Patienten über die Leitungen (302) Zugang erhalt und der Speicher (102) die spezifischen Daten über den Patienten zum externen Verarbeitungssystem zur Anzeige liefert.

**Revendications**

**1.** Ensemble formant cathéter à thermodilution pour mesurer directement les variations de température dans le milieu physiologique d'un patient, l'ensemble comprenant un cathéter à thermodilution (100) ayant un élément chauffant pour chauffer le milieu physiologique,
caractérisé par une mémoire (102) qui se trouve à un endroit prédéterminé sur le cathéter à thermodilution, la mémoire contenant un coefficient d'informations de résistance de l'élément chauffant et des informations de coefficient de transfert de chaleur de l'élément chauffant, pouvant être décodées par un système de traitement externe pour étalonner la résistance et la température de l'élément chauffant.

**2.** Ensemble formant cathéter à thermodilutIon selon la revendication 1, dans lequel la mémoire (12) contient des informations de masse thermique pour l'élément chauffant.

**3.** Ensemble formant cathéter à thermodilution selon la revendication 1 ou 2, dans lequel la mémoire (102) contient des informations de composition filamenteuse pour l'élément chauffant.

**4.** Ensemble formant cathéter à thermodilution selon l'une quelconque des revendications précédentes, dans lequel la mémoire (102) contient des informations spécifiques au patient qui sont accessibles au moyen du système de traitement externe.

**5.** Ensemble formant cathéter à thermodilution selon l'une quelconque des revendications précédentes, dans lequel ledit emplacement prédéterminé se trouve à l'intérieur du corps dudit cathéter (100).

**6.** Ensemble formant cathéter à thermodilution selon l'une quelconque des revendications précédentes, dans lequel ledit emplacement prédéterminé se situe dans une zone adjacente audit élément chauffant.

**7.** Ensemble formant cathéter à thermodilution selon l'une quelconque des revendications précédentes, dans lequel ledit cathéter comprend une thermistance ou un thermocouple pour mesurer les variations de température dans ledit milieu physiologique entraînées par ledit élément chauffant.

**8.** Ensemble formant cathéter à thermodilution selon l'une quelconque des revendications précédentes, comprenant en outre une batterie située d proximité de ladite mémoire (102) pour alimenter ladite mémoire lorsque ladite mémoire n'est pas connectée à un système de traitement externe.

**9.** Ensemble formant cathéter à thermodilution selon l'une quelconque des revendications précédentes, dans lequel ladite mémoire (102) contient en outre des informations d'identification de cathéter comprenant au moins une date de fabrication, un numéro de lot, une date de stérilisation, une date de péremption, un numéro et un type d'élément chauffant ainsi que le nom et l'adresse du fabricant.

**10.** Ensemble formant cathéter à thermodilution selon l'une quelconque des revendications précédentes, comprenant en outre un raccord (300) connecté à une extrémité proximale dudit cathéter à thermodilution pour connecter l'ensemble au système de traitement externe pour, permettre audit élément de chauffage de communiquer avec ledit système de traitement externe, dans lequel ledit emplacement prédéterminé se trouve à l'intérieur dudit raccord (300).

11. Ensemble formant cathéter à thermodilution selon la revendication 10, dans lequel ledit raccord (300) comprend des fils conducteurs (302) qui sont connectés à ladite mémoire (102) afin de permettre l'accès au contenu de la mémoire par ledit système de traitement externe lorsqu'elle est reliée audit ensemble formant cathéter.

12. Ensemble formant cathéter à thermodilution selon l'une quelconque des revendications 1 à 11, dans lequel ladite mémoire (102) contient en outre au moins un programme informatique, un segment de programme informatique, un sous-programme logiciel et des adresses de mémoire informatiques qui peuvent être lus par ledit système de traitement externe et utilisés à cette fin cour vérifier, corriger ou modifier le traitement des informations d'étalonnage de l'élément chauffant.

13. Ensemble formant cathéter à thermodilution selon l'une quelconque des revendications précédentes, dans lequel la mémoire (102) est connectée à un système de traitement externe.

14. Ensemble formant cathéter à thermodilution selon la revendication 13, dans lequel les variations de température mesurées par l'ensemble formant cathéter à thermodilution sont utilisées par ledit système de traitement externe afin, de mesurer le débit cardiaque ou le flux du milieu physiologique dudit patient.

15. Ensemble formant cathéter à thermodilution selon la revendication 13 ou 14 lorsqu'elles dépendent de la revendication 12, dans lequel lesdits fils conducteurs (302) de raccord sont connectés de telle sorte que ledit système de traitement externe puisse écrire les informations d'étalonnage pour ledit patient ou ledit élément chauffant sur ladite mémoire (102) pendant le fonctionnement de l'ensemble formant cathéter pour un étalonnage *in vivo*.

16. Ensemble formant cathéter à thermodilution selon l'une quelconque des revendications 13 à 15 lorsqu'elles dépendent de la revendication 11, dans lequel ledit système de traitement externe accède auxdites informations spécifiques au patient par l'intermédiaire desdits fils conducteurs (302) et ladite mémoire (102) délivre lesdites données spécifiques au patient audit système de traitement externe à des fins d'affichage.

Fig. 1

Fig. 2

CS

104

110

108

CS          VCC
SK  93C46   NC
DI          ORG
DO          GND

102

106

VCC

GND

CLOCK

} HEATER
  CONTROL

DATA IN/OUT

} THERMISTOR
  OUTPUT

EP 0 606 356 B1

Fig.3

Fig.4

Fig.5